# FASCICULE DE BREVET EUROPEEN

(11) **EP 4 110 079 B1**
(45) Date de publication et mention de la délivrance du brevet: **11.11.2026**
(21) Numéro de dépôt: 21714249.6
(22) Date de dépôt: 26.02.2021
(51) Int. Cl.: A23J 3/26, A23P 10/25, A61K 8/64, A61Q 19/00, A23L 11/00, A23J 3/14, A23J 3/22, A61K 36/48, A23L 13/40, A23P 30/20

(54) **COMPOSITION COMPRENANT DES PROTÉINES DE LÉGUMINEUSES TEXTURÉES, SON PROCÉDÉ DE PRODUCTION ET SON UTILISATION**
ZUSAMMENSETZUNG AUS TEXTURIERTEN HÜLSENFRUCHTPROTEINEN, VERFAHREN ZU IHRER HERSTELLUNG UND IHRE VERWENDUNG
COMPOSITION COMPRISING TEXTURED LEGUME PROTEINS, PROCESS FOR ITS PRODUCTION AND ITS USE

(30) Priorité: 28.02.2020 FR 2002039; 07.04.2020 FR 2003484
(43) Date de publication de la demande: 04.01.2023
(73) Titulaire: ROQUETTE FRERES, 62136 Lestrem (FR)
(72) Inventeur: DLUBAK, Charlotte, 62136 LESTREM (FR); BUCHE, Thomas, 62136 LESTREM (FR); DROULEZ, Cyril, 62136 LESTREM (FR); SARRAZIN, Florian, 62136 LESTREM (FR)
(74) Mandataire: Roquette IP International
(86) Numéro de dépôt international: PCT/FR2021/050339
(87) Numéro de publication internationale: WO 2021/170965

(56) Documents cités:
- EP-A1- 2 011 404
- WO-A1-2014/081285
- WO-A2-2009/018548
- US-A1- 2007 269 567
- "A New Dimension to Vegetable Proteins: NUTRALYS® Pea Protein", 1 October 2008 (2008-10-01), XP002803295, Retrieved from the Internet <URL:http://www.fnbnews.com/FB-Specials/A-New-Dimension-to-Vegetable-Proteins-NUTRALYS-Pea-Protein> [retrieved on 20210514]

## Description

La présente invention est relative à une composition spécifique comprenant des protéines de pois texturées, ainsi qu'à son procédé de fabrication et son utilisation.

La technique de texturation des protéines, notamment par cuisson-extrusion, dans le but de préparer des produits à structure fibreuse destinés à la réalisation d'analogues de viande et de poisson, a été appliquée à de nombreuses sources végétales.

On peut séparer en deux grandes familles les procédés de cuisson-extrusion des protéines de par la quantité d'eau mise en œuvre lors du procédé. Lorsque cette quantité est supérieure à 30% en poids, on parlera de cuisson-extrusion dite « humide » et les produits obtenus seront plutôt destinés à la production de produits finis à consommation immédiate, simulant la viande animale par exemple des steaks de bœuf ou bien des nuggets de poulet. On connait par exemple la demande de brevet WO2014081285 qui dévoile un procédé d'extrusion d'un mélange de protéine et de fibres avec utilisation d'une filière de refroidissement (« *cooling die »* en anglais) typique de l'extrusion humide. Notre invention s'inscrit dans le domaine de l'extrusion sèche.

Lorsque cette quantité d'eau est inférieure à 30% en poids, on parle alors de cuisson-extrusion « sèche » : les produits obtenus sont plutôt destinés à être utilisés par les industriels de l'agroalimentaire, afin de formuler des succédanés de viandes, en les mélangeant avec d'autres ingrédients. Le domaine de la présente invention est bien celui de la cuisson-extrusion « sèche ».

Historiquement, les premières protéines utilisées comme analogues de viande ont été extraites du soja et du blé. Le soja est ensuite rapidement devenu la source principale pour ce domaine d'applications.

On connait par exemple la demande de brevet WO2009018548 qui nous enseigne que des mélanges variés contenant des protéines peuvent être extrudés afin de générer une protéine extrudée avec des fibres alignées permettant d'envisager de simuler des fibres de viandes. Pas d'indication cependant sur l'influence de la taille de particule, de la densité ou de la capacité de rétention sur les performances applicatives, ni sur le procédé afin de les produire. La demande de brevet US2007269567 précise les tailles de particules obtenues (11 mm et 16,3 mm en moyenne selon la Table IV de l'exemple 3).

Si la plupart des études qui ont suivi ont naturellement porté sur les protéines de soja, d'autres sources de protéines, tant animales que végétales, ont été texturées : protéines d'arachide, de sésame, de graines de coton, de tournesol, de maïs, de blé, protéines issues de microorganismes, de sous-produits d'abattoirs ou de l'industrie du poisson.

Les protéines de légumineuses telles que celles issues du pois et de la féverole ont fait aussi l'objet de travaux, tant dans le domaine de leur isolement que dans celui de leur cuisson-extrusion « sèche ».

De nombreuses études ont été entreprises sur les protéines de pois, étant donné leurs propriétés fonctionnelles et nutritives particulières, mais aussi pour leur caractère non génétiquement modifié.

Malgré les efforts de recherche importants et une croissance importante au cours de ces dernières années, la pénétration de ces produits à base de protéines texturées sur le marché alimentaire est encore sujette à optimisation.

Une des raisons en particulier tient dans la procédure de réhydratation nécessaire des protéines de pois texturées avant de les formuler.

En effet, celles-ci étant sèches, il est nécessaire de les réhydrater afin de pouvoir les mettre en forme et les mélanger intimement aux autres constituants de la formulation pour obtenir un résultat final satisfaisant.

Pour ce faire, les protéines de pois texturées par voie sèche vont être placées en contact avec une solution aqueuse. Malheureusement, la quantité d'eau absorbée aux fins de réhydratation n'est pas assez efficace et, sans intervention humaine supplémentaire, celle-ci n'est d'environ que de 50% de la quantité nécessaire pour les étapes de formulation suivantes.

Il est donc communément pratiqué une étape supplémentaire dite de « dilacération » (appelé « shredding » en anglais) ou « cuterage » consistant en un hachage des fibres texturées réhydratées. Les fibres ainsi obtenues sont remises en contact avec une solution aqueuse et, du fait du hachage, vont pouvoir réabsorber la quantité d'eau nécessaire manquante.

Cette étape est compliquée car un hachage mal maîtrisé peut endommager les protéines de pois texturées. C'est de plus une étape de préparation supplémentaire qui complexifie la mise en œuvre.

Une solution consiste en une diminution de la taille des particules de protéines texturées, dès l'étape de production. Cette réduction de taille permet d'optimiser la reprise en eau des protéines texturées du fait de la surface d'échange protéines/eau augmentée. L'étape de dilacération après réhydratation devient inutile, du fait de la réduction de taille particulaire réalisée dès la production de la protéine texturée.

Malheureusement, la réduction de la taille de particule des protéines texturées a une conséquence sur les propriétés organoleptiques des analogies de viandes ou de poissons finaux, réalisés avec lesdites protéines végétales texturées. L'article "Effect of soy particle size and color on the sensory properties of ground beef patties" (Cardello & al., Journal of food quality, 1983) présente en sa Figure 3 les conséquences organoleptiques. Cette étude visait à étudier l'impact organoleptique de différentes tailles de protéines de soja texturées dans de la viande de bœuf. On voit bien que les meilleurs résultats sont obtenus, sans atteindre les résultats d'une viande de bœuf, avec les protéines de soja texturées dont la taille de particules supérieure à 9,52 mm représente plus de 73% des particules totales. Toute réduction de cette répartition granulométrique va impliquer une diminution de la reproduction des qualités organoleptiques de l'analogue de viande obtenu.

On peut expliquer cette diminution du résultat organoleptique par une disparition de la quantité et de l'intégrité de la matière nécessaire à émuler les fibres de viandes. Les particules étant plus petites, les fibres obtenues dans l'analogue de viande ou de poisson ne possèdent plus les tailles de fibres effectives suffisantes.

Pour pallier ce problème, une solution potentielle consiste à augmenter la densité des protéines végétales texturées afin de pallier la faible taille de fibres protéiques, par une densification de celles-ci. De courtes fibres protéiques mais plus dense auraient ainsi une structure plus ferme, simulant mieux le résultat organoleptique à atteindre.

Cette stratégie a malheureusement un impact non négligeable sur la capacité de rétention d'eau d'une protéine végétale texturée. L'article « EXTRUSION OF TEXTURIZED PROTEINS » (Kearns & al., American Soybean Association) présente le lien direct établi entre densité et capacité en rétention d'eau (WHC). On peut y voir clairement que la capacité en rétention d'eau chute dès que la densité augmente. Une protéine de soja texturée ayant une densité de 216 g/l possède ainsi une capacité en rétention d'eau à peine supérieure à 3 g d'eau par gramme de protéines, et toujours inférieure à 3,5. Toute augmentation de la densité provoque une chute de cette capacité de rétention d'eau, parfois en dessous de 2.

Cette corrélation négative entre densité et capacité en rétention d'eau est aussi clairement démontrée dans la Table 1 de l'article « Effect of Value-Enhanced Texturized Soy Protein on the Sensory and Cooking Properties of Beef Patties » (A.A. Heywood et al., JAOCS, Vol. 79, no. 7, 2002). Ces données nous confirment donc qu'une haute densité implique une faible capacité de rétention d'eau et inversement. L'obtention d'une protéine texturée ayant à la fois une densité et une rétention d'eau élevées semble donc impossible. Un tel produit est cependant d'intérêt pour l'industrie.

Il est du mérite de la demanderesse d'avoir résolu les problèmes ci-dessus et d'avoir développé une nouvelle composition spécifique comprenant des protéines de légumineuses texturées, obtenue par cuisson-extrusion par voie sèche dont la taille des particules est réduite, la densité est élevée et la capacité de rétention d'eau est améliorée, tout en conservant une protéine texturée donnant d'excellents résultats dans les applications d'analogues de viandes et de poisson.

Cette invention sera mieux comprise dans le chapitre suivant visant à exposer une description générale celle-ci.

### DESCRIPTION GENERALE DE LA PRESENTE INVENTION

La présente invention est relative à une composition comprenant des protéines de légumineuses texturées par voie sèche sous forme de particules, la composition ayant une capacité de rétention d'eau mesurée par un test A supérieure à 3,5 g d'eau par g de protéines sèches, préférentiellement compris entre 3,5 et 4,5 g d'eau par g de protéines sèches, encore plus préférentiellement compris entre 3,5 et 4 g d'eau par g de protéines sèches, une densité mesurée par un test B comprise entre 190 et 230 g/l et au moins 85% des particules de protéines de légumineuses texturées ayant une taille comprise entre 2mm et 5mm.

De manière préférée, la protéine de légumineuse est choisie dans la liste constituée de la féverole et du pois. Le pois est particulièrement préféré.

La teneur en protéines au sein de la composition est comprise entre 60% et 80%, préférentiellement entre 70% et 80% en poids sec par rapport au poids total de matière sèche de la composition.

Enfin, la matière sèche de la protéine de légumineuse texturée par voie sèche selon l'invention est supérieure à 80% en poids, préférentiellement supérieure à 90% en poids.

La présente invention est également relative à un procédé de production d'une composition de protéines de légumineuses telle que décrite ci-dessus caractérisé en ce que le procédé comprend les étapes suivantes :
1) Fourniture d'une poudre comprenant des protéines de légumineuses et des fibres de légumineuses présentant un ratio en poids sec de protéines de légumineuses / fibres de légumineuses compris entre 70/30 et 90/10, préférentiellement compris entre 75/25 et 85/15 ;
2) Cuisson-extrusion de la poudre avec de l'eau, le ratio massique eau/poudre avant cuisson étant compris entre 20% et 40%, préférentiellement entre 25% et 35%, encore plus préférentiellement 30%
3) Coupe de la composition extrudée en sortie d'extrudeuse constituée d'une filière en sortie avec orifices, d'un diamètre de 1,5mm et équipée un couteau dont la vitesse de rotation est comprise entre 1200 et 1800 tours par minutes, ou entre 2000 et 2400 tours par minutes, préférentiellement autour de 1500 tours/min
4) Séchage de la composition ainsi obtenue.

De manière préférée, la protéine de légumineuse mise en œuvre dans le procédé selon l'invention est sélectionnée dans la liste comprenant la féverolle et le pois, préférentiellement une protéine de pois.

La poudre comprenant des protéines de légumineuse et des fibres de légumineuses mise en œuvre à l'étape 1 peut être préparée par mélange desdites protéines et fibres. La poudre peut être constituée essentiellement de protéines de légumineuses et de fibres de légumineuse. Le terme « constitué essentiellement » signifie que la poudre peut comprendre des impuretés liées au procédé de fabrication des protéines et des fibres, telles que par exemple des traces d'amidon. De manière préférée, la protéine et la fibre de légumineuse sont choisies dans la liste composée de la féverole et du pois. Le pois est particulièrement préféré.

De manière préférée, l'étape 2 est réalisée par cuisson-extrusion dans un extrudeur bi-vis caractérisé par un ratio longueur/diamètre compris entre 20 et 45, préférentiellement entre 35 et 45, préférentiellement 40, et équipé d'une 85-95% d'éléments de convoyage, 2,5-10% d'éléments de pétrissage, et 2,5-10% d'éléments de pas inversé.

De manière encore plus préférée, on applique au mélange de poudre une énergie spécifique comprise entre 10 et 25 kWh/kg, en régulant la pression en sortie dans une gamme comprise entre 10 et 25 bars, préférentiellement entre 12 et 16 bars ou entre 17 et 23 bars.

De manière encore plus préférée, la sortie de l'extrudeur bi-vis est constituée d'une filière en sortie avec orifices d'un diamètre de 1,5mm et avec un couteau dont la vitesse de rotation est comprise entre 1200 et 1800 tours par minutes ou entre 2000 et 2400 tours par minutes, préférentiellement 1500 tours/min.

La présente invention est enfin relative à l'utilisation de la composition de protéines de légumineuses texturées par voie sèche telle que décrite ci-dessus dans des applications industrielles telles que par exemple l'industrie alimentaire humaine et animale, la pharmacie industrielle ou la cosmétique.

De manière préférée, la protéine de légumineuse utilisée dans ces applications est une protéine de pois.

La présente invention se comprendra mieux à la lecture de la description détaillée infra.

### DESCRIPTION DETAILLEE DE LA PRESENTE INVENTION

La présente invention est relative à une composition comprenant des protéines de légumineuses texturées par voie sèche sous forme de particules, la composition ayant une capacité de rétention d'eau mesurée par un test A supérieure à 3,5 g d'eau par g de protéines sèches, préférentiellement compris entre 3,5 et 4,5 g d'eau par g de protéines sèches, encore plus préférentiellement compris entre 3,5 et 4 g d'eau par g de protéines sèches , une densité mesurée par un test B comprise entre 190 et 230 g/l et au moins 85% des particules de protéines de légumineuses texturées ayant une taille comprise entre 2mm et 5mm.

De manière préférée, la protéine de légumineuse est choisie dans la liste constituée de la protéine de féverole et de la protéine de pois. La protéine de pois est particulièrement préférée.

Le terme « légumineuses » est considéré ici comme la famille de plantes dicotylédones de l'ordre des Fabales. C'est l'une des plus importantes familles de plantes à fleurs, la troisième après les Orchidaceae et les Asteraceae par le nombre d'espèces. Elle compte environ 765 genres regroupant plus de 19 500 espèces. Plusieurs légumineuses sont d'importantes plantes cultivées parmi lesquelles le soja, les haricots, les pois, la féverole, le pois chiche, l'arachide, la lentille cultivée, la luzerne cultivée, différents trèfles, les fèves, le caroubier, la réglisse.

Le terme « pois » étant ici considéré dans son acception la plus large et incluant en particulier toutes les variétés de « pois lisse » (« *smooth pea* ») et « de pois ridés » (« *wrinkled pea* »), et toutes les variétés mutantes de « pois lisse » et de « pois ridé » et ce, quelles que soient les utilisations auxquelles on destine généralement lesdites variétés (alimentation humaine, nutrition animale et/ou autres utilisations).

Le terme « pois » dans la présente demande inclut les variétés de pois appartenant au genre *Pisum* et plus particulièrement aux espèces *sativum* et *aestivum.* Lesdites variétés mutantes sont notamment celles dénommées « mutants r », « mutants rb », « mutants rug 3 », « mutants rug 4 », « mutants rug 5 » et « mutants lam » tels que décrits dans l'article de C-L HEYDLEY et al. intitulé « Developing novel pea starches » Proceedings of the Symposium of the Industrial Biochemistry and Biotechnology Group of the Biochemical Society, 1996, pp. 77-87.

Si les protéines de légumineuses, en particulier issues de féverolle et de pois, sont particulièrement adaptées à la conception de l'invention, il est néanmoins possible de parvenir à celle-ci avec d'autres sources de protéines végétales telles que les protéines d'avoine, d'haricot mungo, de pomme de terre, de maïs ou encore de pois chiche. L'homme du métier saura faire les adaptations éventuellement nécessaires.

Par « texturée » ou « texturation », on entend dans la présente demande tout procédé physique et/ou chimique visant à modifier une composition comportant des protéines afin de lui conférer une structure ordonnée spécifique. Dans le cadre de l'invention, la texturation des protéines vise à donner l'aspect d'une fibre, telles que présentes dans les viandes animales. Comme il sera décrit dans la suite de cette description, un procédé particulièrement préféré pour texturer les protéines est la cuisson extrusion, particulièrement à l'aide d'un extrudeur bi-vis.

Afin de mesurer la capacité de rétention d'eau, on utilise le test A dont le protocole est décrit ci-dessous :
a. Peser 20g d'échantillon à analyser dans un bécher
b. Ajouter de l'eau potable à température ambiante (température entre 10°c et 20°C, préférentiellement 20°C +/- 1°C) jusqu'à submersion complète de l'échantillon ;
c. Laisser en contact statique pendant 30 minutes ;
d. Laisser égoutter ;
e. Séparer eau résiduelle et échantillon à l'aide d'un tamis ;
f. Peser le poids final P de l'échantillon réhydraté ;

Le calcul de la Capacité de rétention d'eau, exprimée en gramme d'eau par gramme de protéine analysée est le suivant : $Capacité de Rétention en eau = \left(P-20\right) / 20 .$

Par « eau potable » on entend une eau que l'on peut boire ou utiliser à des fins domestiques et industrielles sans risque pour la santé. De manière préfèrentielle, sa conductivité est choisie entre 400 et 1100, préférentiellement entre 400 et 600 µS/cm. De manière plus préférentielle dans la présente invention, on entendra que cette eau potable possède une teneur en sulfate inférieure à 250 mg/l, une teneur en chlorures inférieure à 200 mg/l, une teneur en potassium inférieure à 12 mg/l, un pH compris entre 6,5 et 9 et un TH (Titre Hydrométrique, soit la dureté de l'eau, qui correspond à la mesure de la teneur d'une eau en ions calcium et magnésium) supérieur à 15 degrés français. Autrement dit, une eau potable ne doit pas posséder moins de 60 mg/l de calcium ou 36 mg/l de magnésium.

Afin de mesurer la densité, on utilise le test B dont le protocole est décrit ci-dessous :
a. Tare d'une éprouvette graduée de 2 litres ;
b. Remplissage de l'éprouvette avec le produit à analyser, jusqu'à atteindre la graduation de 2 litres.
c. Pesée du produit (Poids P, en grammes).

Le calcul de la densité exprimée en g/l est le suivant : $Densité = \left(P\left(en g\right)/2\right)$

Le protocole pour déterminer la taille des particules constitutives mesurée selon un test C, exprimée en pourcentage est le suivant :
- On utilise un système de tamis empilés sur une machine permettant de mettre en agitation lesdits tamis, afin de faire circuler les particules au travers des mailles. Une référence commerciale particulièrement adaptée est la suivante Tamisseuse Electromagnétique de laboratoire, modèle Analysette 3, commercialisé par la société FRITSCH.
Les différents tamis utilisés sont les suivants 1mm, 2 mm, 5 mm, 10 mm
- On introduit 100g de produit au sommet et on met l'appareillage en mode vibration pendant 3 min. On peut modifier ce temps, tant que l'on s'assure que la séparation granulométrique est bien terminée.
- Après arrêt, on pèse le poids de chaque fraction accumulée sur chaque tamis que l'on appelle «le refus » du tamis. C'est en effet les particules n'ayant pas réussi à passer la maille car trop gros.
- Le calcul est le suivant : $Supérieur à 10 mm = \left(poids refus 10 mm/poids X\right) * 100$ $Entre 5 et 10 mm = \left(poids refus 5 mm/Poids X\right) * 100$ $Entre 2 et 5 mm = \left(poids refus 2 mm/Poids X\right) * 100$ $Entre 1 et 2 mm = \left(poids refus 1 mm/Poids X\right) * 100$ $Inférieur à 1 mm = \left(poids refus final/Poids X\right) * 100$

Comme indiqué ci-dessus, les compositions de protéine de pois texturées de l'art antérieur sont déjà bien connues et utilisées dans l'industrie alimentaire, en particulier dans les analogues de viande. Afin de les mettre en œuvre dans une recette, il est connu que la teneur en eau nécessaire est de minimum 3g par g de protéines, 4 g étant préférée. Cette réhydratation va permettre de préparer les fibres à être incluses dans la formulation, en simulant au mieux les propriétés fonctionnelles de fibres de viande, et éviter la présence trop importante de parties mal réhydratées provoquant une sensation de dureté, de croustillance lors de la consommation finale. Il est aussi connu que cette réhydratation ne peut être réalisée en une seule étape.

L'homme du métier, connaissant la difficulté de reprise en eau des protéines texturées, pratique tout d'abord à une première réhydratation en plaçant la protéine de pois texturée avec un solvant aqueux, en atteignant environ 2g d'eau par g de protéines. Puis, il procédera à une dilacération des fibres de protéines réhydratées. Sans être lié par une quelconque théorie, cette dilacération (ou « shredding » en anglais) va permettre de déstructurer les fibres et ouvrir ainsi les parties internes et permettre leur réhydratation. Il suffira donc de replacer les fibres de protéines réhydratées et déstructurées en contact avec du solvant aqueux, la capacité de rétention en eau sera supérieure à 3,5 g par g de protéines.

On retrouve par exemple l'indication de cette nécessité de l'étape de dilacération sur la documentation technique du NUTRALYS^{®} T70S produit et commercialisé par la demanderesse (cf l'extrait « *Recipe preparation includes a shredding step of NUTRALYS^{®} T70S* » cité dans le lien https://www.roquette.com/- /media/contenus-gbu/food/plant-proteins---concepts/roquette-food-breakfast-sausage-us-2020-04-1511-(1).pdf).

La dilacération (ou « shredding » en anglais) des protéines est une solution bien connue mais elle rajoute une étape, complexifiant le procédé de formulation final, engendrant une augmentation des coûts. De plus, cette dilacération si elle est mal maitrisée va provoquer une déstructuration trop importante des fibres, provoquant une perte des effets fonctionnels recherchés. Les fibres végétales en ayant été raccourcies simuleront moins bien les fibres de viande.

Enfin, la matière sèche de la protéine de légumineuse texturée par voie sèche selon l'invention est supérieure à 80% en poids, préférentiellement supérieure à 90% en poids.

La matière sèche est mesurée par toute méthode bien connue de l'homme de l'art. De manière préférentielle, la méthode dite « par dessication » est utilisée. Elle consiste à déterminer la quantité d'eau évaporée par chauffage d'une quantité connue d'un échantillon de masse connue. Le chauffage est continu jusqu'à stabilisation de la masse, indiquant que l'évaporation de l'eau est complète. De manière préférée, la température utilisée est de 105°C.

La teneur en protéine de la composition selon l'invention est avantageusement comprise entre 60% et 80%, préférentiellement entre 70% et 80% en poids sur la matière sèche totale. Pour analyser cette teneur en protéines, n'importe quelle méthode bien connue par l'homme du métier est utilisable. De préférence, on dosera la quantité d'azote total et l'on multipliera cette teneur par le coefficient 6,25. Cette méthode est particulièrement connue et utilisée pour les protéines végétales.

La présente invention est également relative à un procédé de production d'une composition de protéines de légumineuses telle que décrite ci-dessus caractérisé en ce que le procédé comprend les étapes suivantes :
1) Fourniture d'une poudre comprenant des protéines de légumineuses et des fibres de légumineuses présentant un ratio en poids sec de protéines de légumineuses / fibres de légumineuses compris entre 70/30 et 90/10, préférentiellement compris entre 75/25 et 85/15 ;
2) Cuisson-extrusion de la poudre avec de l'eau, le ratio massique eau/poudre avant cuisson étant compris entre 20% et 40%, préférentiellement entre 25% et 35%, encore plus préférentiellement 30%
3) Séchage de la composition ainsi obtenue.

De manière préférée, la protéine de légumineuses et la fibre de légumineuses de l'étape 1 sont choisies dans la liste constituée de la protéine de féverole et de la protéine de pois. La protéine de pois est particulièrement préférée.

La poudre comprenant des protéines de légumineuses et des fibres de légumineuses mise en œuvre à l'étape 1 peut être préparée par mélange desdites protéines et fibres. La poudre peut être constituée essentiellement de protéines de légumineuses et de fibres de légumineuses. Le terme « constitué essentiellement » signifie que la poudre peut comprendre des impuretés liées au procédé de fabrication des protéines et des fibres, telles que par exemple des traces d'amidon. Le mélange consiste à obtenir un mélange sec des différents constituants nécessaires à synthétiser la fibre végétale lors de l'étape 2.

De manière préférée, les protéines de légumineuses sont caractérisées par une teneur en protéines avantageusement comprise entre 60% et 90%, préférentiellement entre 70% et 85%, encore plus préférentiellement entre 75% et 85% en poids sur la matière sèche totale. Pour analyser cette teneur en protéines, n'importe quelle méthode bien connue par l'homme du métier est utilisable. De préférence, on dosera la quantité d'azote total et l'on multipliera cette teneur par le coefficient 6,25. Cette méthode est particulièrement connue et utilisée pour les protéines végétales. De manière préférée, la matière sèche de la protéine de légumineuse est supérieure à 80% en poids, préférentiellement supérieure à 90% en poids.

De manière encore plus préférée, les protéines de légumineuses sont caractérisées par une solubilité à pH 3 supérieure à 30%. La solubilité est mesurée à l'aide du protocole suivant : on réalise une suspension de la poudre à 2,5% p/p avec de l'eau distillée avec une quantité Q1, on rectifie le pH à la valeur désirée, on agite 30 min à 1100 tr/min à l'aide d'un barreau magnétique, on centrifuge 15 min à 3000g puis on analyse la quantité de matière Q2 dans le surnageant à l'aide de son poids et de sa matière sèche (obtenue p.ex. par la méthode dite « par dessication ». Elle consiste à déterminer la quantité d'eau évaporée par chauffage d'une quantité connue d'un échantillon de masse connue. Le chauffage est continu jusqu'à stabilisation de la masse, indiquant que l'évaporation de l'eau est complète. De manière préférée, la température utilisée est de 105°C.). La solubilité est obtenue par la formule : (Q2 / Q1 ) * 100d

De manière encore plus préférée, les protéines sont caractérisées par une granulométrie caractérisée par un Dmode compris entre 150 microns et 400 microns, préférentiellement entre 150 microns et 200 microns ou entre 350 microns et 450 microns. La mesure de cette granulométrie est réalisée à l'aide d'un granulomètre laser MALVERN 3000 en phase sèche (équipé d'un module poudre). La poudre est placée dans l'alimentation du module avec une ouverture comprise entre 1 et 4mm et une fréquence de vibration de 50% ou 75. L'appareil enregistre automatiquement les différentes tailles et restitue la Distribution de Taille des Particules (ou PSD en anglais) ainsi que le Dmode, le D10, le D50 et le D90. Le Dmode est bien connu de l'Homme du Métier consiste en la taille de la population de particules la plus importante.

La granulométrie de la poudre est avantageuse pour la stabilité et la productivité du procédé. Une granulométrie trop fine est irrémédiablement suivie de problèmes parfois lourds à gérer lors du procédé d'extrusion.

Par « fibres de légumineuses », on entend toutes compositions comportant des polysaccharides peu ou non digestibles par le système digestif humain, extraites de légumineuses. De telles fibres sont extraites par tout procédé bien connu de l'homme du métier.

De manière préférée, la fibre de légumineuse est issue du pois à l'aide d'un procédé d'extraction par voie humide. Le pois dépelliculé est réduit en farine qui est ensuite mis en suspension dans de l'eau. La suspension ainsi obtenue est envoyée sur des hydrocyclones afin d'extraire l'amidon. Le surnageant est envoyé dans des décanteurs horizontaux afin d'obtenir une fraction fibre de légumineuse. Un tel procédé est décrit dans la demande de brevet EP2950662. Une fibre de légumineuse ainsi préparée contient entre 40% et 60% de polymères composés de cellulose, d'hémicellulose et de pectine, préférentiellement entre 45% et 55%, ainsi qu'entre 25% et 45% d'amidon de pois, préférentiellement entre 30% et 40%. Un exemple commercial d'une telle fibre est par exemple la fibre Pea Fiber I50 de la société Roquette.

Le mélange peut être réalisé en amont à l'aide d'un mélangeur à sec ou bien directement en alimentation de l'étape 2. Lors de ce mélange, on peut ajouter des additifs bien connus de l'homme du métier tels que des arômes ou bien des colorants.

Dans un mode alternatif, le mélange fibre/protéines est naturellement obtenu par turboséparation d'une farine de légumineuses. Les graines de légumineuses sont nettoyées, débarrassées de leurs fibres externes et broyées en farine. La farine est ensuite turboséparée, ce qui consiste en l'application d'un courant d'air ascendant permettant une séparation des différentes particules selon leur densité. On arrive ainsi à concentrer la teneur en protéines dans les farines d'environ 20% à plus de 60%. De telles farines sont appelées « concentrats ». Ces concentrats contiennent également entre 10% et 20% de fibres de légumineuses.

Le ratio massique sec entre protéines et fibres est avantageusement compris entre 70/30 et 90/10, préférentiellement compris entre 75/25 et 85/15.

Lors de l'étape 2, ce mélange de poudres va ensuite être texturé ce qui revient à dire que les protéines et les fibres vont subir une déstructuration thermique et une réorganisation afin de former des fibres, un allongement continu en lignes droites parallèles, simulant les fibres présentes dans les viandes. Tout procédé bien connu de l'homme du métier conviendra, en particulier par extrusion.

L'extrusion consiste à forcer un produit à s'écouler à travers un orifice de petite dimension, la filière, sous l'action de pressions et de forces de cisaillements élevées, grâce à la rotation d'une ou deux vis d'Archimède. L'échauffement qui en résulte provoque une cuisson et/ou dénaturation du produit d'où le terme parfois utilisé de "cuisson-extrusion", puis une expansion par évaporation de l'eau en sortie de filière. Cette technique permet d'élaborer des produits extrêmement divers dans leur composition, leur structure (forme expansée et alvéolée du produit) et leurs propriétés fonctionnelles et nutritionnelles (dénaturation des facteurs antinutritionnels ou toxiques, stérilisation des aliments par exemple). Le traitement de protéines conduit souvent à des modifications structurelles qui se traduisent par l'obtention de produits à l'aspect fibreux, simulant les fibres de viandes animales.

L'étape 2 doit être réalisée avec un ratio massique eau/poudre avant cuisson étant compris entre 20% et 40%, préférentiellement entre 25% et 35%, encore plus préférentiellement 30%. Ce ratio est obtenu en divisant la quantité d'eau par la quantité de poudre, et en multipliant par 100. De manière préférée, l'eau est injectée à la fin de la zone de convoyage et juste avant la zone de pétrissage.

Sans être lié par une quelconque théorie, il est bien connu de l'homme du métier de la cuisson extrusion que c'est ce ratio qui permettra d'obtenir la densité requise. Les valeurs de ce ratio seront donc potentiellement 20, 21, 22, 23, 24, 25, 26, 27, 28, 29, 30, 31, 32, 33, 34, 35, 36, 37, 38, 39 ou 40%.

Toute eau dite potable convient pour ce faire. Par « eau potable » on entend une eau que l'on peut boire ou utiliser à des fins domestiques et industrielles sans risque pour la santé. De manière préférentielle, sa conductivité est choisie entre 400 et 1100, préférentiellement entre 400 et 600 µS/cm. De manière plus préférentielle dans la présente invention, on entendra que cette eau potable possède une teneur en sulfate inférieure à 250 mg/l, une teneur en chlorures inférieure à 200 mg/l, une teneur en potassium inférieure à 12 mg/l, un pH compris entre 6,5 et 9 et un TH (Titre Hydrométrique, soit la dureté de l'eau, qui correspond à la mesure de la teneur d'une eau en ions calcium et magnésium) supérieur à 15 degrés français. Autrement dit, une eau potable ne doit pas posséder moins de 60 mg/l de calcium ou 36 mg/l de magnésium. Cette définition inclus l'eau du réseau potable, l'eau décarbonatée, l'eau déminéralisée.

De manière préférée, l'étape 2 est réalisée par cuisson-extrusion dans un extrudeur bi-vis caractérisé par un ratio longueur/diamètre compris entre 20 et 45, préférentiellement entre 35 et 45, préférentiellement 40, et équipé d'une succession de 85-95% d'éléments de convoyage, 2,5-10% d'éléments de pétrissage, et 2,5-10% d'éléments de pas inversé.

Le ratio longueur/diamètre est un paramètre classique dans la cuisson-extrusion. Ce ratio pourra donc être de 20, 21, 22, 23, 24, 25, 26, 27, 28, 29, 30, 31, 32, 33, 34, 35, 36, 37, 38, 39, 40, 41, 42, 43, 44 ou 45.

Les différents éléments sont les éléments de convoyage visant à convoyer le produit dans la filière sans modifier le produit, les éléments de pétrissage visant à mélanger le produit et les éléments de pas inversé visant à appliquer une force au produit pour le faire progresser à contre-sens et ainsi provoquer mélange et cisaillement.

De manière préférée, les éléments de convoyage seront placés en tout début de vis avec une température réglée entre 20°C et 70°C, puis les éléments de pétrissage avec une température comprise entre 90°C et 150°C et enfin les éléments de pas inversés avec des températures comprises entre 100°C et 120°c.

De manière préférée, cette vis est mise en rotation entre 900 et 1200 tours/min, préférentiellement entre 1000 et 1100 tours/min.

De manière encore plus préférée, on applique au mélange de poudre une énergie spécifique comprise entre 10 et 25 kWh/kg, en régulant la pression en sortie dans une gamme comprise entre 10 et 25 bars, préférentiellement entre 12 et 16 bars ou entre 17 et 23 bars.

L'étape 3 consiste ensuite en une coupe de la composition extrudée en sortie d'extrudeuse constituée d'une filière en sortie avec orifices, d'un diamètre de 1,5mm et équipée un couteau dont la vitesse de rotation est comprise entre 1200 et 1800 tours par minutes, ou entre 2000 et 2400 tours par minutes, préférentiellement autour de 1500 tours/min.

Le couteau est placé à fleur de la sortie de l'extrudeuse, préférentiellement à une distance comprise entre 0 et 5mm. Par « fleur » on entend à une distance extrêmement proche de la filière située à la sortie de l'extrudeuse, à la limite de toucher la filière mais sans toucher celle-ci. De manière classique, l'homme du métier réglera cette distance en faisant se toucher le couteau et la filière, puis en décalant très légèrement celle-ci.

La dernière étape 4 consiste au séchage de la composition ainsi obtenue.

L'homme du métier saura utiliser la technologie adéquate afin de sécher la composition selon l'invention dans le vaste choix qui lui est actuellement offert. On peut citer sans limitation et à seule fin d'exemplification les séchoirs à flux d'air, les séchoirs à micro-ondes, les séchoirs à lit fluidisés ou les séchoirs sous vide. Il sélectionnera les bons paramètres, principalement temps et température, afin d'atteindre la matière sèche finale désirée.

La présente invention est enfin relative à l'utilisation de la composition de protéines de légumineuses texturées par voie sèche telle que décrite supra dans des applications industrielles telles que par exemple l'industrie alimentaire humaine et animale, la pharmacie industrielle ou la cosmétique.

Par industrie alimentaire humaine et animale, on entend la confiserie industrielle (par exemple chocolat, caramel, bonbons gélifiés), les produits de boulangerie-pâtisserie (par exemple le pain, les brioches, les muffins), l'industrie de la viande et du poisson (par exemple les saucisses, les steak-hachés, les nuggets de poisson, les nuggets de poulet), les sauces (par exemple bolognaise, mayonnaise), les produits dérivés du lait (par exemple fromage, lait végétal), les boissons (par exemple boissons riches en protéines, boissons en poudre à reconstituer).

De manière plus préférée, la présente invention est relative à l'utilisation de la composition de protéines de légumineuses texturées par voie sèche telle que décrite supra dans le domaine de la boulangerie-pâtisserie.

L'invention sera particulièrement d'intérêt afin de réaliser des inclusions dans des produits de boulangerie-pâtisserie tels que muffins, cookies, cakes, bagel, pâte à pizza, pains et céréales pour le petit-déjeuner.

Par « inclusions », on entend des particules (ici la composition de protéines de légumineuses texturées par voie sèche) mélangées avec une pâte avant sa cuisson. Après celle-ci, la composition de protéines de légumineuses texturées par voie sèche est piégée dans le produit final (d'où le terme « inclusion ») et apportent à la fois sa teneur en protéine ainsi qu'un caractère croustillant lors de la consommation.

L'invention sera particulièrement d'intérêt afin de réaliser des inclusions dans des produits de confiserie tels que fat filings, chocolats, de manière à apporter également une tenue en protéines ainsi qu'un caractère croustillant.

L'invention sera particulièrement d'intérêt afin de réaliser des inclusions dans des produits alternatifs aux produits laitiers tels que fromages, yaourts, glaces et boissons.

L'invention sera particulièrement d'intérêt dans le domaine des analogues de viandes, de poissons, de sauces, de soupes.

Une application particulière concerne l'utilisation de la composition selon l'invention pour la fabrication de substitut de viande, notamment de viande hachée. Mais également sauce bolognaise, steak pour hamburger, viande pour tacos et pitta, « chili sin carne ».

Dans les pizzas, la composition comprenant des protéines de légumineuses texturées selon l'invention sera particulièrement d'intérêt pour être saupoudrée au-dessus de la dite pizza (« topping » en anglais).

Dans les plats cuisinés déshydratés (par exemple. Bolino en Europe ou Good Dot en Inde), on utilisera la composition texturée selon l'invention en tant qu'élément apportant de fibreux et de protéine. Ainsi, il est possible d'obtenir un produit qui s'hydrate vite et jusqu'à son cœur tout en apportant une mâche intéressante.

L'invention sera mieux comprise à la lecture des exemples non limitatifs ci-dessous.

### Exemples

### Exemple 1 : Production d'une composition de protéines de légumineuses texturées par voie sèche selon l'invention

On réalise un mélange poudre constitué de 87% de protéine de pois NUTRALYS^{®} F85M (comportant 87,2% de protéines) de la société ROQUETTE et 12,5% de fibre de pois I50M. La teneur en protéines dans 100g de mélange est donc de 87* 0,872 = 75,9g.

Ce mélange est introduit par gravité dans un extrudeur COPERION ZSK 54 MV de la société COPERION.

Le mélange est introduit avec un débit régulé de 300 kg/h. Une quantité de 78 kg/h d'eau est également introduite. Le ratio massique eau/poudre est donc de (78 / 300 ) * 100 = 26%.

La vis d'extrusion, composée de 85 % d'éléments de convoyage, 5% d'éléments de pétrissage et 10% d'éléments à pas inversé, est mise en rotation à une vitesse de 1000 tours/min et envoie le mélange dans une filière. Comme indiqué dans la description, les éléments de convoyage ont été placés en tout début de vis avec une température réglée entre 20°C et 70°C, puis les éléments de pétrissage avec une température comprise entre 90°C et 150°C et enfin les éléments de pas inversés avec des températures comprises entre 100°C et 120°C.

Cette conduite particulière génère un couple machine de 41% avec une pression en sortie de 20 bars. L'énergie spécifique du système est d'environ 17 KWh/Kg
Le produit est dirigé en sortie vers une filière constituée de 44 trous cylindrique de 1,5 mm, d'où est expulsée la protéine texturée qui est coupée à l'aide de couteaux tournant à 1500 tours / minutes placés à fleur de la sortie de la filière d'extrusion.

La protéine texturée ainsi produite est séchée dans un séchoir VD 14 x 14 KM*1 de marque Geelen Counterflow à une température de 88°C dans un flux d'air chaud de 2400 kg/h.

Une mesure de capacité en rétention d'eau selon le test A nous indique une valeur de 3,8 g/g d'eau.

Une mesure de densité de la protéine extrudée à l'aide du test B nous indique une valeur de 210 g/L.

### Exemple 2 : Production d'une composition de protéines de légumineuses texturées par voie sèche hors invention (Ratio eau/MS trop bas)

On réalise un mélange poudre constitué de 87% de protéine de pois NUTRALYS^{®} F85M (comportant 87,2% de protéines) de la société ROQUETTE et 12,5% de fibre de pois I50M.

Ce mélange est introduit par gravité dans un extrudeur COPERION ZSK 54 MV de la société COPERION.

Le mélange est introduit avec un débit régulé de 300 kg/h. Une quantité de 55 kg/h d'eau est également introduite. Le ratio massique eau/poudre est donc de (55 / 300) * 100 = 18,3%.

La vis d'extrusion, composée de 85 % d'éléments de convoyage, 5% d'éléments de pétrissage et 10% d'éléments à pas inversé, est mise en rotation à une vitesse comprise à 575 tours/min et envoie le mélange dans une filière. Comme indiqué dans la description, les éléments de convoyage ont été placés en tout début de vis avec une température réglée entre 20°C et 70°C, puis les éléments de pétrissage avec une température comprise entre 90°C et 150°C et enfin les éléments de pas inversés avec des températures comprises entre 100°C et 120°c.

Cette conduite particulière génère un couple machine de 65% avec une pression en sortie de 25 bars. L'énergie spécifique du système est d'environ 14 KWh/Kg.

Le produit est dirigé en sortie vers une filière constituée de 44 trous cylindrique de 1,5 mm, d'où est expulsée la protéine texturée qui est coupée à l'aide de couteaux tournant à 2100 tours / minutes.

La protéine texturée ainsi produite est séchée dans un séchoir Dryer VD 14 x 14 KM*1 à une température de 86°C dans un flux d'air chaud de 2000 kg/h.

Une mesure de capacité en rétention d'eau selon le test A nous indique une valeur de 3,4 g/g d'eau.

Une mesure de densité de la protéine extrudée à l'aide du test B nous indique une valeur de 115g/L.

Un essai supplémentaire a été réalisé avec les mêmes paramètres mais la vitesse de vis a été augmentée à 1075 tours/min : la densité était encore plus basse, à 103 g/L.

### Exemple 2 bis : Production d'une composition de protéines de légumineuses texturées par voie sèche hors invention (ratio eau/MS trop haut)

On réalise un mélange poudre constitué de 87% de protéine de pois NUTRALYS^{®} F85M (comportant 87,2% de protéines) de la société ROQUETTE et 12,5% de fibre de pois I50M.

Ce mélange est introduit par gravité dans un extrudeur COPERION ZSK 54 MV de la société COPERION.

Le mélange est introduit avec un débit régulé de 300 kg/h. Une quantité de 130 kg/h d'eau est également introduite. Le ratio massique eau/poudre est donc de (55 / 300 ) * 100 = 43,3%.

La vis d'extrusion, composée de 85 % d'éléments de convoyage, 5% d'éléments de pétrissage et 10% d'éléments à pas inversé, est mise en rotation à une vitesse comprise à 575 tours/min et envoie le mélange dans une filière. Comme indiqué dans la description, les éléments de convoyage ont été placés en tout début de vis avec une température réglée entre 20°C et 70°C, puis les éléments de pétrissage avec une température comprise entre 90°C et 150°C et enfin les éléments de pas inversés avec des températures comprises entre 100°C et 120°c.

Cette conduite particulière génère un couple machine de 35% avec une pression en sortie de 15 bars.

Le produit est dirigé en sortie vers une filière constituée de 44 trous cylindrique de 1,5 mm, d'où est expulsée la protéine texturée qui est coupée à l'aide de couteaux tournant à 2100 tours / minutes.

La protéine texturée ainsi produite est séchée dans un séchoir Dryer VD 14 x 14 KM*1 à une température de 86°C dans un flux d'air chaud de 2000 kg/h.

Une mesure de capacité en rétention d'eau selon le test A nous indique une valeur de 1,5 g/g d'eau.

Une mesure de densité de la protéine extrudée à l'aide du test B nous indique une valeur de 301 g/L.

### Exemple 3 : Production d'une composition de protéines de légumineuses texturées par voie sèche hors invention (ratio fibres/protéines trop bas)

On réalise un mélange poudre constitué de 99% de protéine de pois NUTRALYS^{®} F85M (comportant 87,5% de protéines) de la société ROQUETTE et 1% de fibre de pois I50M. La teneur en protéines dans 100g de mélange est donc de 99 * 0,80 = 79,2g.

Ce mélange est introduit par gravité dans un extrudeur COPERION ZSK 54 MV de la société COPERION.

Le mélange est introduit avec un débit régulé de 300 kg/h. Une quantité de 78 kg/h d'eau est également introduite. Le ratio massique eau/poudre est donc de (78 / 300) * 100 = 26%.

La vis d'extrusion, composée de 85 % d'éléments de convoyage, 5% d'éléments de pétrissage et 10% d'éléments à pas inversé, est mise en rotation à une vitesse comprise à 1000 tours/min et envoie le mélange dans une filière. Comme indiqué dans la description, les éléments de convoyage ont été placés en tout début de vis avec une température réglée entre 20°C et 70°C, puis les éléments de pétrissage avec une température comprise entre 90°C et 150°C et enfin les éléments de pas inversés avec des températures comprises entre 100°C et 120°c.

Cette conduite particulière génère un couple machine de 40% avec une pression en sortie de 19bars.

Le produit est dirigé en sortie vers une filière constituée de 44 trous cylindrique de 1,5 mm, d'où est expulsée la protéine texturée qui est coupée à l'aide de couteaux tournant à 1500 tours / minutes placés à fleur de la sortie de la filière d'extrusion.

La protéine texturée ainsi produite est séchée dans un séchoir VD 14 x 14 KM*1 de marque Geelen Counterflow à une température de 88°C dans un flux d'air chaud de 2400 kg/h.

Une mesure de capacité en rétention d'eau selon le test A nous indique une valeur de 3,4 g/g d'eau.

Une mesure de densité de la protéine extrudée à l'aide du test B nous indique une valeur de 105 g/L.

### Exemple 4 : Production d'une composition de protéines de légumineuses texturées par voie sèche (exemple vitesse de coupe plus inférieure)

On réalise un mélange poudre constitué de 87,5% de protéine de pois NUTRALYS^{®} F85M (comportant 80% de protéines) de la société ROQUETTE et 12,5% de fibre de pois I50M. La teneur en protéines dans 100g de mélange est donc de 87,5 * 0,80 = 70g.

Ce mélange est introduit par gravité dans un extrudeur COPERION ZSK 54 MV de la société COPERION.

Le mélange est introduit avec un débit régulé de 300 kg/h. Une quantité de 78 kg/h d'eau est également introduite. Le ratio massique eau/poudre est donc de (78 / 300) * 100 = 26%.

La vis d'extrusion, composée de 85 % d'éléments de convoyage, 5% d'éléments de pétrissage et 10% d'éléments à pas inversé, est mise en rotation à une vitesse comprise à 1000 tours/min et envoie le mélange dans une filière. Comme indiqué dans la description, les éléments de convoyage ont été placés en tout début de vis avec une température réglée entre 20°C et 70°C, puis les éléments de pétrissage avec une température comprise entre 90°C et 150°C et enfin les éléments de pas inversés avec des températures comprises entre 100°C et 120°c.

Cette conduite particulière génère un couple machine de 60% avec une pression en sortie de 23 bars.

Le produit est dirigé en sortie vers une filière constituée de 44 trous cylindrique de 1,5 mm, d'où est expulsée la protéine texturée qui est coupée à l'aide de couteaux tournant à 500 tours / minutes placés à fleur de la sortie de la filière d'extrusion.

La protéine texturée ainsi produite est séchée dans un séchoir VD 14 x 14 KM*1 de marque Geelen Counterflow à une température de 88°C dans un flux d'air chaud de 2400 kg/h.

Une mesure de capacité en rétention d'eau selon le test A nous indique une valeur de 3,8 g/g d'eau.

Une mesure de densité de la protéine extrudée à l'aide du test B nous indique une valeur de 209 g/L.

### Exemple 5 : Comparaison des compositions de protéines de légumineuses texturées par voie sèche obtenues dans les exemples ci-dessus et de compositions issues de l'art antérieur

On met en œuvre les protocoles décrits dans la partie supra de la description, afin de mesurer la densité selon le test B, la capacité de rétention en eau selon le test A ainsi que la taille des particules constitutives mesurée selon un test C.

On compare les échantillons obtenus dans les exemples 1 à 4, mais également une sélection de protéines texturées du marché.

**[Tableau 1]**

| Ex. | Humidité (en %/poids) | Densité (g/l) | Capacité rétention eau (g/g) | % taille 5 à 10 mm | % taille 2 à 5 mm | % taille 0 à 2 mm |
|---|---|---|---|---|---|---|
| Exemple 1 Selon invention | 10,1 | 210 | 3,8 | 0,4 | 90,7 | 8,8 |
| Exemple 2 Ratio eau/MS inférieur | 7,5 | 115 | 3,4 | 8,8 | 79 | 13,3 |
| Exemple 2 bis Ratio Eau/MS supérieur | 8,1 | 301 | 1,5 | non déterminé | | |
| Exemple 3 Ratio fibres/protéines inférieur | 8,5 | 105 | 3,4 | non déterminé | | |
| Exemple 4 Vitesse de coupe inférieure | 8,6 | 209 | 3,8 | 24 | 73,6 | 2,2 |
| Nutralys T70S (Roquette, pois) | 8,2 | 120 | 2,5 | 75 | 10 | 3 |
| Bona Vita (Sojovi Granulat, soja) | 10 | 300 | 3,4 | 17 | 73 | 9 |
| Trutex (MGP, soja) | 9 | 260 | 2,5 | 25 | 61 | 14 |

On s'aperçoit donc que seul le produit selon l'exemple 1 permet d'obtenir une composition dont la Capacité de Rétention d'eau selon le test A est supérieure à 3,5 g d'eau par gramme de protéines sèches. La composition de l'exemple 1 est unique, car élevée en capacité de rétention d'eau mais avec une densité supérieure à 200 g/l. Par ailleurs, la répartition granulométrique est satisfaisante en ce qu'au moins 85% de particules ont une taille entre 2 et 5 mm.

### Exemple 6 : Mise en œuvre d'une composition de protéines de légumineuses texturées par voie sèche selon l'invention dans des analogues de viandes

On procède à la réalisation d'un steak-haché ou burger mettant en œuvre les compositions présentées dans les exemples.

Les ingrédients mis en œuvre sont les suivants (les quantités indiquées dans le tableau ci-dessous sont données en grammes pour 100g de burger final):

**[Tableau 2]**

| Ingrédients | Recette burger #1 |
|---|---|
| Eau potable | 53,55 |
| Protéine texturée | 19,5 |
| Glace pilée | 6 |
| Methylcellulose | 2 |
| Oignons | 5,9 |
| Huile de tournesol | 5,4 |
| Fécule de pomme de terre native | 2 |
| Fibre Pea Fiber I50 (Roquette) | 3 |
| Poudre d'ail | 0,5 |
| Sel | 0,2 |
| Poivre noir | 0,1 |

La procédure de production est la suivante :
1. Hydrater les protéines texturées dans de l'eau potable pendant 30 min
2. Uniquement pour le burger avec le NUTRALYS T70S (hors invention - ligne 3 du tableau 1), broyer pendant 45s le mélange protéines texturées/eau à l'aide d'un robot mixer KENWOOD FDM302SS (vitesse 1), puis laisser de nouveau 30 min en contact avec de l'eau
3. Mélanger du methylcellulose et de la glace pilée dans un récipient, puis réserver 5 min au réfrigérateur.
4. Mélanger dans un autre récipient l'ensemble des autres ingrédients
5. Réunir dans un même récipient les mélanges obtenus aux étapes 1 (voire 2), 3 et 4 et mélanger afin d'obtenir une composition homogène.
6. Former manuellement des steaks hachés avec le mélange final d'une quantité d'environ 150 g

Après dégustation par un panel de 10 personnes, il est reconnu que le burger réalisé avec la protéine texturée selon l'invention est plus proche d'un burger à base de viande animale qu'un burger réalisé avec le NUTRALYS^{®} T70S : la sensation fibreuse y est plus présente lors de la dégustation, moins caoutchouteux.

Il est très étonnant du fait des connaissances antérieures (cf paragraphe 18 faisant référence à l'article « *Effect of soy particle size and color on the sensory properties of ground beef patties »*) d'obtenir un meilleur résultat organoleptique avec la protéine texturée selon l'invention qui possède une taille de particules plus petite que la protéine de pois texturée NUTRALYS^{®} T70S. C'est la sélection précise et particulière des caractéristiques de capacité de rétention d'eau et de densité qui permet d'obtenir cet excellent résultat avec cette petite taille de particule et sans l'étape de dilacération.

Le panel juge majoritairement que le burger obtenu avec la protéine texturée selon l'exemple 3 donne un résultat plus mou, plus caoutchouteux, et donc plus éloigné qu'avec la protéine selon l'invention.

Le panel juge aussi majoritairement que le burger obtenu obtenue avec la protéine texturée selon l'exemple 4 donne un aspect extérieur plus différent que le contrôle, en faisant apparaitre de plus grosses particules.

### Exemple 7 : Mise en œuvre d'une composition de protéines de légumineuses texturées par voie sèche selon l'invention dans une sauce bolognaise :

On procède à la réalisation d'une sauce bolognaise mettant en œuvre les compositions présentées dans les exemples.

Les ingrédients mis en œuvre sont les suivants (les quantités indiquées dans le tableau 3 ci-dessous sont données en grammes pour 100g de sauce finale):

**[Tableau 3]**

| Ingrédients | Recette sauce bolognaise |
|---|---|
| Eau potable | 56,10 |
| Protéine texturée | 5,5 |
| Extrait de pomme | 0,16 |
| Concentré de tomates | 33,2 |
| Vinaigre | 0,83 |
| Sel | 0,91 |
| Amidon CLEARAM^{®} CH3020 (ROQUETTE) | 1,82 |
| Herbes de provence | 0,3 |

La procédure de production est la suivante :
1. Mélanger tous les ingrédients dans un HotmixPro Creative
2. Cuire à 90°C pendant 10min à vitesse 2
3. Remplir un pot de conserve avec la sauce obtenue
4. Stériliser 1 heure à 120°C avec un stérilisateur Steriflow^{®}

Un exemple comparatif a été réalisé. Selon cet exemple comparatif, la protéine texturée selon l'invention est remplacée par NUTRALYS T70S dans la recette de sauce bolognaise ci-dessus.

Après dégustation par un panel de 10 personnes, il est reconnu que la sauce bolognaise réalisée avec la protéine texturée selon l'invention est plus proche d'une sauce bolognaise à base de viande animale qu'une sauce bolognaise réalisée avec le NUTRALYS T70S : à la dégustation, on sent moins la présence de grosses particules.

Le panel juge majoritairement que la sauce bolognaise obtenue avec la protéine texturée selon l'exemple 4 donne un résultat plus éloigné qu'avec la protéine texturée selon l'invention car la sensation de grosses particules y est plus importante.

### Exemple 8 : Mise en œuvre d'une composition de protéines de légumineuses texturées par voie sèche selon l'invention pour produire une saucisse végétale :

On procède à la réalisation d'une saucisse végétale mettant en œuvre les compositions présentées dans les exemples.

Les ingrédients mis en œuvre sont les suivants (les quantités indiquées dans le tableau 4 ci-dessous sont données en grammes pour 100g de saucisse finale):

**[Tableau 4]**

| Ingrédients | Recette saucisse #1 |
|---|---|
| Eau potable | 50,02 |
| Protéine texturée | 17,47 |
| Blanc d'œuf | 4,48 |
| Fibre de pois I50M (ROQUETTE) | 0,91 |
| Amidon de pomme de terre natif (ROQUETTE) | 1,73 |
| Amidon de pomme de terre PREGEFLO P100 (ROQUETTE) | 0,91 |
| Gluten de blé (ROQUETTE) | 1,73 |
| Miettes de pain | 1,73 |
| Isolat de protéine pois NUTRALYS^{®} F85F (ROQUETTE) | 0,91 |
| Hule de tournesol | 7,79 |
| Cubes de poivrons rouges 10*10 | 8,81 |
| Oignons éminçés | 0,96 |

La procédure de production est la suivante :
1. D'un côté, hydrater la composition de protéine texturée selon l'invention pendant 30 min dans l'eau
2. D'un autre côté, mélanger toutes les poudres ensemble
3. Ajouter les deux mélanges ci-dessus dans le bol d'un Kenwood, avec également l'huile de tournesol, les poivrons et l'oignon.
5. Mélanger 3 minutes à vitesse 1
6. Introduire le mélange dans des boyaux artificiels
7. Refroidir dans de l'eau fraiche (10°C) puis peler les boyaux artificiels

Un exemple comparatif a été réalisé. Selon cet exemple comparatif, la protéine texturée selon l'invention est remplacée par NUTRALYS T70S dans la recette de saucisse ci-dessus.

Après dégustation par un panel de 10 personnes, il est reconnu que la saucisse réalisée avec la protéine texturée selon l'invention est plus proche d'une saucisse à base de viande animale qu'une saucisse réalisée avec le NUTRALYS T70S : à la dégustation la composition interne est beaucoup plus homogène.

De même que l'exemple précédent, il est très étonnant du fait des connaissances antérieures (cf paragraphe 18 faisant référence à l'article « *Effect of soy particle size and color on the sensory properties of ground beef patties »*) d'obtenir un meilleur résultat organoleptique avec la protéine texturée selon l'invention qui possède une taille de particules plus petite que la protéine de pois texturée NUTRALYS^{®} T70S. C'est la sélection précise et particulière des caractéristiques de capacité de rétention d'eau et de densité qui permet d'obtenir cet excellent résultat avec cette petite taille de particules et sans l'étape de dilacération.

### Exemple 9 : Mise en œuvre d'une composition de protéines de légumineuses texturées par voie sèche selon l'invention pour produire des muesli croustillants (ou « crunchy clusters » en anglais):

On procède à la réalisation de muesli croustillants mettant en œuvre les compositions présentées dans les exemples.

Les ingrédients mis en œuvre sont les suivants (les quantités indiquées dans le tableau 5 ci-dessous sont données en grammes pour 100g de saucisse finale):

**[Tableau 5]**

| Ingrédients (en g) | Recette contrôle | Recette avec protéines de pois texturées de l'exemple 1 | Recette avec protéines de pois texturées de l'art antérieur (NUTRALYS^{®} T70S) |
|---|---|---|---|
| Flocons d'avoine (Quaker Oats) | 40 | 28 | 28 |
| Riz soufflé (Rice Krispies, Kellogs) | 10 | | |
| Flocons de maîs (Corn-Flakes, Kellog's) | 10 | 10 | 10 |
| Protéine de pois texturée | | 22 | 22 |
| Saccharose | 17 | | |
| Eau | 10 | | |
| Huile de tournesol | 8 | | |
| Sirop de glucose (Glucose syrup 6080, ROQUETTE) | 5 | | |
| Total | 100 | 100 | 100 |

La procédure de production est la suivante :
1. Mélanger le saccharose, l'eau, le sirop de glucose et l'huile afin de préparer un sirop en chauffant et agitant à l'aide d'un Hotmix, vitesse 2 à 85°C(le poids peut être vérifié pour éviter / corriger toute évaporation d'eau)
2. Ajouter les autres ingrédients et mélanger à vitesse 1 à l'aide d'un Kitchen'Aid Artisan 5KSM175PS
3. Étaler sur une plaque à pâtisserie et cuire à 140 ° C pendant 25 minutes

Après dégustation par un panel de 10 personnes, il est reconnu que les mueslis croustillants réalisée avec la protéine texturée selon l'invention est plus proche des muesli croustillants contrôles qu'un muesli croustillants réalisé avec le NUTRALYS T70S. En effet, les différents ingrédients du cluster sont jugés être liés de manière plus lâche avec NUTRALYS^{®} T70S.

Le panel juge majoritairement que les mueslis croustillants obtenus avec la protéine texturée selon l'exemple 4 sont jugés également être liés de manière plus lâche.

## Revendications

1. Composition comprenant des protéines de légumineuses texturées par voie sèche sous forme de particules, la composition ayant une capacité de rétention d'eau mesurée par un test A supérieure à 3,5 g d'eau par g de protéines sèches, préférentiellement compris entre 3,5 et 4,5 g d'eau par g de protéines sèches , encore plus préférentiellement compris entre 3,5 et 4 g d'eau par g de protéines sèches, une densité mesurée par un test B comprise entre 190 et 230 g/l et au moins 85% des particules de protéines de légumineuses texturées ayant une taille comprise entre 2mm et 5mm.

2. Composition de protéines de légumineuses texturées par voie sèche selon la revendication 1 **caractérisée en ce que** la protéine de légumineuse est choisie dans la liste constituée de la protéine de féverole et de la protéine de pois.

3. Composition de protéines de légumineuses texturées par voie sèche selon les revendications 1 et 2 **caractérisée en ce que** la teneur en protéines au sein de la composition est comprise entre 60% et 80%, préférentiellement entre 70% et 80% en poids sec.

4. Composition de protéines de légumineuses texturées par voie sèche selon l'une des revendications 1 à 3 **caractérisée en ce qu'**elle présente une matière sèche supérieure à 80% en poids, préférentiellement supérieure à 90% en poids.

5. Procédé de production d'une composition comprenant de protéines de légumineuses selon l'une des revendications 1 à 4, le procédé est **caractérisé en ce qu'**il comprend les étapes suivantes :
1) Fourniture d'une poudre comprenant des protéines de légumineuses et des fibres de légumineuses présentant un ratio en poids sec de protéines de légumineuses / fibres de légumineuses compris entre 70/30 et 90/10, préférentiellement compris entre 75/25 et 85/15 ;
2) Cuisson-extrusion de la poudre avec de l'eau, le ratio massique eau/poudre avant cuisson étant compris entre 20% et 40%, préférentiellement entre 25% et 35%, encore plus préférentiellement 30% ;
3) Coupe de la composition extrudée en sortie d'extrudeuse constituée d'une filière en sortie avec orifices, d'un diamètre de 1,5mm et équipée un couteau dont la vitesse de rotation est comprise entre 1200 et 1800 tours par minutes, ou entre 2000 et 2400 tours par minutes, préférentiellement autour de 1500 tours/min ;
4) Séchage de la composition ainsi obtenue.

6. Procédé de production selon la revendication 5, **caractérisé en ce que** la protéine de légumineuse est une protéine de pois.

7. Procédé de production selon la revendication 6 **caractérisée en ce que caractérisée en ce que** la protéine de pois possède une teneur en protéines avantageusement comprise entre 60% et 90%, préférentiellement entre 70% et 85%, encore plus préférentiellement entre 75% et 85% en poids sur la matière sèche totale.

8. Procédé de production selon les revendications 6 ou 7, **caractérisé en ce que** la protéine de pois est **caractérisée par** une granulométrie **caractérisée par** un Dmode compris entre 150 microns et 400 microns, préférentiellement entre 150 microns et 200 microns ou entre 350 microns et 450 microns.

9. Procédé de production selon l'une des revendications 5 à 8, **caractérisé en ce que** la fibre de légumineuse contient entre 40% et 60% de polymères composés de cellulose, d'hémicellulose et de pectine, préférentiellement entre 45% et 55%, ainsi qu'entre 25% et 45% d'amidon de pois, préférentiellement entre 30% et 40%.

10. Procédé de production selon l'une des revendication 5 à 9, **caractérisé en ce que** l'étape 2 est réalisée par cuisson-extrusion dans un extrudeur bi-vis **caractérisé par** un ratio longueur/diamètre compris entre 35 et 45, préférentiellement 40, et équipé d'une succession de 85-95% d'éléments de convoyage, 2,5-10% d'éléments de pétrissage, et 2,5-10% d'éléments de pas inversé.

11. Procédé de production selon la revendication 10 **caractérisée en ce que** les éléments de convoyage seront placés en tout début de vis avec une température réglée entre 20°C et 70°C, puis les éléments de pétrissage avec une température comprise entre 90°C et 150°C et enfin les éléments de pas inversés avec des températures comprises entre 100°C et 120°C.

12. Procédé de production selon l'une des revendications 10 ou 11 **caractérisée en ce que** la vis est mise en rotation entre 900 et 1200 tours/min, préférentiellement entre 1000 et 1100 tours/min.

13. Procédé de production selon l'une des revendications 5 à 12, **caractérisé en ce qu'**est appliqué au mélange de poudre une énergie spécifique comprise entre 10 et 25 kWh/kg, en régulant la pression en sortie dans une gamme comprise entre 10 et 25 bars, préférentiellement entre 12 et 16 bars.

14. Utilisation d'une composition de protéines de légumineuses texturées par voie sèche selon l'une des revendications 1 à 4 ou produite selon le procédé décrit dans l'une des revendications 5 à 13 dans une application industrielle choisie parmi l'industrie alimentaire humaine et animale, la pharmacie industrielle ou la cosmétique.

15. Utilisation selon la revendication 14, **caractérisée en ce que** la protéine de légumineuse est une protéine de pois.

## Patentansprüche

1. Zusammensetzung, umfassend trocken texturierte Leguminosenproteine in Form von Partikeln, wobei die Zusammensetzung ein durch einen Test A gemessenes Wasserhaltevermögen von mehr als 3,5 g Wasser pro g trockener Proteine, vorzugsweise zwischen 3,5 und 4,5 g Wasser pro g trockener Proteine, noch mehr bevorzugt zwischen 3,5 und 4 g Wasser pro g trockener Proteine, eine durch einen Test B gemessene Dichte zwischen 190 und 230 g/l aufweist und mindestens 85 % der Partikel aus texturierten Leguminosenproteinen eine Größe zwischen 2 mm und 5 mm aufweisen.

2. Zusammensetzung trocken texturierter Leguminosenproteine nach Anspruch 1, **dadurch gekennzeichnet, dass** das Leguminosenprotein ausgewählt ist aus der Liste bestehend aus Ackerbohnenprotein und Erbsenprotein.

3. Zusammensetzung trocken texturierter Leguminosenproteine nach den Ansprüchen 1 und 2, **dadurch gekennzeichnet, dass** der Proteingehalt in der Zusammensetzung zwischen 60 % und 80 %, vorzugsweise zwischen 70 und 80 Gew.-%, bezogen auf die Trockenmasse liegt.

4. Zusammensetzung trocken texturierter Leguminosenproteine nach einem der Ansprüche 1 bis 3, **dadurch gekennzeichnet, dass** sie eine Trockenmasse von mehr als 80 Gew.-%, vorzugsweise von mehr als 90 Gew.-%, aufweist.

5. Herstellungsverfahren einer Zusammensetzung, umfassend Leguminosenproteine, nach einem der Ansprüche 1 bis 4, wobei das Verfahren **dadurch gekennzeichnet ist, dass** es die folgenden Schritte umfasst:
1) Bereitstellen eines Pulvers, umfassend Leguminosenproteine und Leguminosenfasern mit einem Trockengewichtsverhältnis von Leguminosenproteinen / Leguminosenfasern zwischen 70/30 und 90/10, vorzugsweise zwischen 75/25 und 85/15;
2) Kochextrusion des Pulvers mit Wasser, wobei das Massenverhältnis von Wasser/Pulver vor dem Kochen zwischen 20 % und 40 % liegt, vorzugsweise zwischen 25 % und 35 %, noch mehr bevorzugt 30 %;
3) Schneiden der extrudierten Zusammensetzung am Extruderausgang, bestehend aus einer Ausgangsdüse mit Öffnungen mit einem Durchmesser von 1,5 mm und ausgestattet mit einem Messer, dessen Drehzahl zwischen 1200 und 1800 Umdrehungen pro Minute oder zwischen 2000 und 2400 Umdrehungen pro Minute liegt, vorzugsweise bei etwa 1500 U/min;
4) Trocknen der auf diesem Wege erhaltenen Zusammensetzung.

6. Herstellungsverfahren nach Anspruch 5, **dadurch gekennzeichnet, dass** das Leguminosenprotein ein Erbsenprotein ist.

7. Herstellungsverfahren nach Anspruch 6, **dadurch gekennzeichnet, dass** das Erbsenprotein einen Proteingehalt vorteilhafterweise zwischen 60 Gew.-% und 90 Gew.-%, vorzugsweise zwischen 70 Gew.-% und 85 Gew.-%, noch mehr bevorzugt zwischen 75 Gew.-% und 85 Gew.-%, bezogen auf die Gesamttrockenmasse, aufweist.

8. Herstellungsverfahren nach Anspruch 6 oder 7,
**dadurch gekennzeichnet, dass** das Erbsenprotein durch eine Partikelgröße gekennzeichnet ist, die durch einen Dmode zwischen 150 Mikrometern und 400 Mikrometern, vorzugsweise zwischen 150 Mikrometern und 200 Mikrometern oder zwischen 350 Mikrometern und 450 Mikrometern, gekennzeichnet ist.

9. Herstellungsverfahren nach einem der Ansprüche 5 bis 8,
**dadurch gekennzeichnet, dass** die Leguminosenfaser zwischen 40 % und 60 % Polymere, die aus Cellulose, Hemicellulose und Pektin bestehen, vorzugsweise zwischen 45 % und 55 %, sowie zwischen 25 % und 45 % Erbsenstärke, vorzugsweise zwischen 30 % und 40 %, enthält.

10. Herstellungsverfahren nach einem der Ansprüche 5 bis 9,
**dadurch gekennzeichnet, dass** Schritt 2 durch Kochextrusion in einem Doppelschneckenextruder durchgeführt wird, **gekennzeichnet durch** ein Längen-/Durchmesserverhältnis zwischen 35 und 45, vorzugsweise 40, und ausgestattet mit einer Abfolge von 85-95 % Förderelementen, 2,5-10 % Knetelementen und 2,5-10 % Elementen mit umgekehrter Abstufung.

11. Herstellungsverfahren nach Anspruch 10,
**dadurch gekennzeichnet, dass** die Förderelemente ganz am Anfang der Schnecke mit einer zwischen 20 °C und 70 °C eingestellten Temperatur angeordnet werden, dann die Knetelemente mit einer Temperatur zwischen 90 °C und 150 °C und schließlich die Elemente mit umgekehrter Abfolge mit Temperaturen zwischen 100 °C und 120 °C.

12. Herstellungsverfahren nach einem der Ansprüche 10 oder 11,
**dadurch gekennzeichnet, dass** die Schnecke zwischen 900 und 1200 U/min, vorzugsweise zwischen 1000 und 1100 U/min, in Drehung versetzt wird.

13. Herstellungsverfahren nach einem der Ansprüche 5 bis 12,
**dadurch gekennzeichnet, dass** auf die Pulvermischung eine spezifische Energie zwischen 10 und 25 kWh/kg aufgebracht wird, wobei der Ausgangsdruck in einem Bereich zwischen 10 und 25 bar, vorzugsweise zwischen 12 und 16 bar, geregelt wird.

14. Verwendung einer Zusammensetzung von trocken texturierten Leguminosenproteinen nach einem der Ansprüche 1 bis 4 oder einer nach dem in einem der Ansprüche 5 bis 13 beschriebenen Verfahren hergestellten Zusammensetzung in einer industriellen Anwendung, ausgewählt aus der Lebensmittel- und Futtermittelindustrie, der industriellen Pharmazie oder der Kosmetik.

15. Verwendung nach Anspruch 14, **dadurch gekennzeichnet, dass** das Leguminosenprotein ein Erbsenprotein ist.

## Claims

1. A composition comprising leguminous proteins textured in a dry process in the form of particles, the composition having a water holding capacity measured by a test A of more than 3.5 g of water per g of dry proteins, preferably ranging between 3.5 and 4.5 g of water per g of dry proteins, even more preferably ranging between 3.5 and 4 g of water per g of dry proteins, a density measured by a test B ranging between 190 and 230 g/l and at least 85% of the textured leguminous protein particles being between 2 mm and 5 mm in size.

2. The composition of leguminous proteins textured in a dry process according to claim 1, **characterized in that** the leguminous protein is selected from the list consisting of faba bean protein and pea protein.

3. The composition of leguminous proteins textured in a dry process according to claims 1 and 2, **characterized in that** the protein content of the composition ranges between 60% and 80%, preferably between 70% and 80% by dry weight.

4. The composition of leguminous proteins textured in a dry process according to any of claims 1 to 3, **characterized in that** it has a dry matter content of more than 80% by weight, preferably of more than 90% by weight.

5. A method for producing a composition comprising leguminous proteins according to any of claims 1 to 4, the method is **characterized in that** it comprises the following steps:
1) providing a powder comprising leguminous proteins and leguminous fibers, having a dry weight ratio of leguminous proteins to leguminous fibers ranging between 70/30 and 90/10, preferably ranging between 75/25 and 85/15;
2) extrusion cooking the powder with water, the water to powder mass ratio before cooking ranging between 20% and 40%, preferably between 25% and 35%, even more preferably 30%;
3) cutting the extruded composition at the extruder outlet made up of an outlet die with holes, with a diameter of 1.5 mm and equipped with a knife, the speed of rotation of which ranges between 1,200 and 1,800 revolutions per minute, or between 2,000 and 2,400 revolutions per minute, preferably around 1,500 revolutions per minute;
4) drying the composition thus obtained.

6. The production method according to claim 5, **characterized in that** the leguminous protein is a pea protein.

7. The production method according to claim 6, **characterized in that** the pea protein has a protein content advantageously ranging between 60% and 90%, preferably between 70% and 85%, even more preferably between 75% and 85% by weight of the total dry matter.

8. The production method according to claims 6 or 7, **characterized in that** the pea protein is **characterized by** a particle size **characterized by** a Dmode ranging between 150 microns and 400 microns, preferably between 150 microns and 200 microns or between 350 microns and 450 microns.

9. The production method according to any of claims 5 to 8, **characterized in that** the leguminous fiber contains between 40% and 60% of polymers made up of cellulose, hemicellulose and pectin, preferably between 45% and 55%, as well as between 25% and 45% of pea starch, preferably between 30% and 40%.

10. The production method according to any of claims 5 to 9,
**characterized in that** step 2 is carried out by extrusion cooking in a twin-screw extruder **characterized by** a length to diameter ratio ranging between 35 and 45, preferably 40, and equipped with a series of 85-95% feeding elements, 2.5-10% kneading elements, and 2.5-10% reverse pitch elements.

11. The production method according to claim 10, **characterized in that** the feeding elements will be placed at the very beginning of the screw with a temperature set between 20°C and 70°C, then the kneading elements with a temperature ranging between 90°C and 150°C and finally the reverse pitch elements with temperatures ranging between 100°C and 120°C.

12. The production method according to any of claims 10 or 11,
**characterized in that** the screw is rotated between 900 and 1,200 revolutions per minute, preferably between 1,000 and 1,100 revolutions per minute.

13. The production method according to any of claims 5 to 12,
**characterized in that** a specific power ranging between 10 and 25 kWh/kg is applied to the powder mixture, by regulating the pressure at the outlet in a range ranging between 10 and 25 bars, preferably between 12 and 16 bars.

14. Use of a composition of leguminous proteins textured in a dry process according to any of claims 1 to 4, or produced according to the method described in any of claims 5 to 13, in an industrial application selected from the human and animal food industry, industrial pharmaceuticals or cosmetics.

15. The use according to claim 14, **characterized in that** the leguminous protein is a pea protein.
